# EUROPEAN PATENT APPLICATION

(11) **EP 1 457 522 A1**
(43) Date of publication of application: **15.09.2004**
(21) Application number: 04251476.0
(22) Date of filing: 15.03.2004
(51) Int. Cl.: C08L 1/28, A23G 3/30, C11B 9/00

(54) **Cellulose-based particles or liquids and methods for their preparation and use**

(30) Priority: 13.03.2003 US 388113; 24.10.2003 US 693527
(71) Applicant: INTERNATIONAL FLAVORS & FRAGRANCES INC., New York New York 10019 (US)
(72) Inventor: Popplewell, Lewis Michael, Morganville New Jersey 07751 (US); Bryant, Cory Michael, Washington D.C. 20009 (US); Henson, Lulu S., Plainsboro New Jersey 08536 (US)
(74) Representative: Mercer, Christopher Paul

(57) **Abstract**

A particle composition containing a continuous phase of 0.5-95% organically soluble cellulosic material selected from the group consisting of ethyl cellulose and hydroxypropyl cellulose dissolved in 5-99.5% organic fragrance chemicals and organic flavor chemicals. The particles are formed from a continuous phase product that results from dissolving the cellulosic material in the flavor/fragrance which is subsequently subjected to techniques to form the desired particle size. Methods of making and using the particles are also disclosed.

## Description

### FIELD OF INVENTION

The invention relates to compositions containing organically compatible cellulosic materials, specifically, ethyl cellulose and hydroxypropyl cellulose that are capable of forming substantially continuous solutions with flavors and fragrances, particles formed from these compositions, as well as methods for the preparation and use of such compositions.

### DESCRIPTION OF THE RELATED ART

Cellulose is a natural polymer. In fact, cellulose, a major component of the plant cell wall, is the most abundant polysaccharide in nature. Cellulose has been modified in many ways to achieve differing results. Some modifications improve water solubility, others improve organic solubility, still other modifications adapt cellulose for special purposes such as for use as a hemostat as in the case of oxidized cellulose.

In the pharmaceutical industry, cellulose materials are often used as binder material in tablets. Cellulose materials have also been modified to achieve desired solubility characteristics for use in sustained or controlled release coatings. Release of encapsulated products is controlled by varying the type and amount of cellulosic material, affecting the rate of dissolution in the body.

In the food industry, cellulose materials have been used in small amounts, generally under 2%, to increase viscosity and body in aqueous systems. Amounts over 2% generally lead to inappropriate viscosities. Products having more than 2% cellulosic materials tend to be thick, difficult to manage substances. These products are plagued by difficulty in handling and especially clean-up.

In recent years, the food, cosmetics, and fragrance industries have found interest in encapsulated products. Such products allow incorporation of flavor or fragrance in small distinct packages that can be dispersed throughout a dispersion medium. Such encapsulated products are generally formed from carriers or encapsulants that are not miscible or compatible with the active agent. For example, hydrophobic substances are often used to surround and encapsulate hydrophilic substances. This type of particle is a core-shell type particle, or in some cases a multi-core type particle. As suggested by the name, two discrete phases make up the particle. The core and shell are each maintained as individual components rather than a continuous phase.

El-Nokaly in U.S. 5,599,555 reveals the use of modified celluloses, among other materials, for encapsulation via liquid crystal formation with an appropriate non-active solvent for the cellulose.

A continuing challenge in the area of flavor/fragrance delivery, then, is the identification of carriers that provide release characteristics suitable for a specific application and environment.

### SUMMARY OF THE INVENTION

The composition comprises a continuous phase of 0.5-95% organically soluble cellulosic material selected from the group consisting of ethyl cellulose and hydroxypropyl cellulose dissolved in 5-99.5% organic fragrance chemicals and/or organic flavor chemicals. The particles are formed from a continuous phase product that results from dissolving the cellulosic material in the flavor/fragrance which is subsequently subjected to size reduction by which the desired particle size is obtained. Methods of making and using the particles are also disclosed.

Materials selected from the group of triglycerides, monoglycerides, diglycerides, and propylene glycol may also be added to aid in particle creation and alter release characteristics.

Materials that are not soluble in the continuous flavor/fragrance plus cellulosic phase may also be added to aid in particle creation and alter release characteristics. These materials may also be flavors or fragrances in a form essentially insoluble in the continuous phase.

### BRIEF DESCRIPTION OF THE DRAWINGS

Figure 1 is a depiction of an extruded particle of the cellulosic material with the flavor/fragrance dissolved throughout the particle.
Figure 2 is a depiction of an extruded particle of the cellulosic material with the solvent and flavor/fragrance dissolved throughout the particle. The particle also depicts particles containing additive particles.

### DETAILED DESCRIPTION

Certain preferred embodiments of the particles and methods of producing them are described below. Those skilled in the art will recognize that other variants of these embodiments are possible without departing from the scope and spirit of the invention.

Organically modified celluloses, and particularly ethyl cellulose and hydroxypropyl cellulose, are soluble at high levels in many traditional mixtures of flavor and/or fragrance chemicals, in some cases with the addition of heat. Once dissolved and cooled, the mixtures are normally stable, substantially smooth and uniform solutions, showing no sign of precipitation. These mixtures typically demonstrate increased viscosity, which may be easily controlled via the amount and type of modified cellulose added. Viscosity may be increased to a point where a firm, hard solid is produced, although this is not required, and sometimes not desirable. The mixture allows for the delivery of flavor and/or fragrance materials by either influencing diffusion or by maintaining physical integrity, again, depending upon need.

The type and content of cellulosic material is in large part determined by the desired characteristics of the microparticle, and its ultimate end use. Some common benefits achieved to various degrees depending upon the cellulosic material content are delayed or prolonged flavor/fragrance release, improved stability, and improvement in handling characteristics.

The consistency of the particles can vary over a wide range. The particles can vary from a viscous material to a solid material. Those with skill in the art will appreciate that a liquid is defined by a disordered or random structure with the disordered dissolved state throughout the solvent. These systems are also called isotropic solutions. This is contrast to a solid material that has a specific or defined structure, usually referred to as a crystalline structure. The present invention does not contemplate the liquid crystalline structures that are defined as having well defined viscosity and light birefringence properties as described more fully in U.S. Patent 5,599,555.

Accordingly the viscosity of the continuous phase of the particle varies over a wide range. Viscosity of the continuous phase ranges from about 100 mPas to about 1,000,000 mPas; preferably from about 20,000 mPas to about 200,000 mPas. As used herein viscosity is measured using an oscillatory rheometer.

Particles of the invention generally contain from about 0.5 to about 95% cellulosic material, and from about 5 to about 99.5% organic flavor and/or fragrance chemicals. In addition, optional additives such as solid or liquid bulking carriers, solid flavors/fragrances, fillers, and other functional materials may be provided in the particles. The level of the additives is from about 1 to about 95, preferably form about 5 to about 80 and most preferably from about 10 to about 60 weight percent of the particle.

Cellulosic material as used herein means those organically modified polymeric celluloses that are organically soluble. These particular celluloses may have dual solubility in both organic solvents and water, but must be soluble at least in organic solvents. This is necessary since the flavor and/or fragrance is organic in nature. Celluloses with dual solubility have interesting properties that make them preferred in certain embodiments. Ethyl cellulose which is only soluble in organic solvents and hydroxypropyl cellulose which is soluble in both organic solvents and water, are the currently preferred cellulosic materials.

Suitable solvents for use with ethylcellulose or hydroxypropyl cellulose include but are not limited to those limited in DOW Chemical Bulletin 192-00818-398GIN and HERCULES Bulletin 250-2F Rev. 10-01 500, respectively. Other modified celluloses, such as methylcellulose and hydroxypropyl methyl cellulose, which are water soluble, will not function in the methods and compositions described herein because they are not generally soluble in the flavor and/or fragrance chemicals. It should be noted that methylcellulose and hydroxypropyl methyl cellulose could be used in a similar way if a solvent compatible with both the specific flavor/fragrance and the polymer were used in a sufficient amount to create a continuous phase between the solvent, polymer, and flavor/fragrance. The inventors have successfully used hydroxypropyl cellulose marketed under the brand name KLUCEL® distributed by the Aqualon division of Hercules Incorporated, and ethylcellulose marketed under the brand ETHOCEL® by DOW Chemical Company.

In a highly preferred embodiment of the invention triglyceride oil is added. The preferred triglyceride is NEOBEE® M5 as sold by Stepan Chemical. The level of triglyceride ranges from about 0.1 to about 70 weight percent of the particles, preferably from about 1 to about 50 and most preferably from about 5 to about 30 weight percent of the particles. Those with skill in the art recognize that many flavor and fragrance materials are sold in a compatible solvent base. The present invention contemplates the use of these fragrance and flavor materials as received, with the use of triglyceride as a solvent for the flavor and/or fragrance chemicals, and as a mode of increasing the hydrophobicity of the particles.

The reduction of solvents is advantageous in that it reduces any emission to the atmosphere and lower solvent particles are easier to form and process. The level of solvent, other than triglyceride and flavor and fragrance material, is preferably below 20 weight percent, more preferably less than about 10 weight percent and most preferably less than about 5 weight percent of the particle. As noted above in highly preferred embodiments of the invention there is no intentionally added solvents, not including triglycerides, flavor and fragrance materials.

Flavor and/or fragrance chemicals are commonly liquid organic solutions that normally contain a variety of constituents varying in chemical class, as well as physical and chemical characteristics. In addition to active flavor and fragrance compounds, these mixtures often employ a compatible solvent that serves to ensure that a uniform, robust solution is formed. This solvent, which is often a triglyceride oil, may normally represent up to 70% of the mixture by weight. In the invention discussed here, this solvent is not a necessary part of forming the desired particles, but is rather a diluent/bulking agent added for convenience or for development of specific physical/chemical properties. It should be noted also that at the temperatures used in forming the particles of the invention, normally less than 110°C, and preferably less than 90°C, triglyceride oil does not solvate either ethylcellulose or hydroxypropyl cellulose appreciably. Rather, the flavor/fragrance materials are the solvent for the modified celluloses.

In addition to the flavor oil and the cellulose material in a preferred embodiment an emulsifier is also included. Suitable food grade emulsifiers are well-known in the art and are described in U.S. Patents 4,479,969 and 6,190,705, the contents of which are incorporated by reference. More specifically the emulsifiers include mono and di-glycerol esters of fatty acids, polyglycerol esters, and sorbitol esters. A preferred emulsifier for the present invention is CAPMUL MCM (ABITEC Corp., Columbus, Ohio). CAPMUL MCM is a mono-diglyceride of medium chain fatty acids (mainly caprylic and capric). Other emulsion stabilizers can also be used without departing from the scope of the present invention.

Many types of flavor and fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in U.S. Patent No. 4,534,891, the contents of which are incorporated by reference as if set forth in its entirety. Another source of suitable fragrances is found in Fragrances, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

As used herein olfactory effective amount is understood to mean the amount of compound in fragrance compositions the individual component will contribute to its particular olfactory characteristics, but the olfactory effect of the fragrance composition will be the sum of the effects of each of the fragrance or fragrance ingredients. Thus the compounds of the invention can be used to alter the aroma characteristics of the fragrance composition by modifying the olfactory reaction contributed by another ingredient in the composition. The amount will vary depending on many factors including other ingredients, their relative amounts and the effect that is desired.

The level of compound of the invention employed in the fragranced article varies from about 0.005 to about 10 weight percent, preferably from about 0.1 to about 8 and most preferably from about 0.5 to about 5 weight percent. In addition to the compounds, other agents can be used in conjunction with the fragrance. Well known materials such as surfactants, emulsifiers, and polymers to encapsulate the fragrance can also be employed without departing from the scope of the present invention. Those with skill in the art will be able to employ the desired level of the compound of the invention to provide the desired fragrance and intensity.

As used herein flavor effective amount is understood to mean the amount of compound in flavor compositions the individual component will contribute to its particular olfactory characteristics, but the flavor effect on the composition will be the sum of the effects of each of the flavor ingredients. Thus the compounds of the invention can be used to alter the taste characteristics of the flavor composition by modifying the taste reaction contributed by another ingredient in the composition. The amount will vary depending on many factors including other ingredients, their relative amounts and the effect that is desired.

The level of flavor ingredient employed in the food can vary widely. The level of most flavor ingredients employed is greater than 100 parts per trillion, generally provided at a level of from about 1 parts per million to 2% in the finished food or confectionary product, more preferably and generally from about 10 parts per million to about 100 parts per million.

The term "foodstuff" as used herein includes both solid and liquid ingestible materials for man or animals, which materials usually do, but need not, have nutritional value. Thus, foodstuffs include meats, gravies, soups, convenience foods, malt, alcoholic and other beverages, milk and dairy products, seafoods, including fish, crustaceans, mollusks and the like, candies, vegetables, cereals, soft drinks, snacks, baked goods, dog and cat foods, other veterinary products and the like. Chewing gum is also included, as are oral care products.

When the compounds of this invention are used in a flavoring composition, they can be combined with conventional flavoring materials or adjuvants. Such co-ingredients or flavor adjuvants are well known in the art for such use and have been extensively described in the literature. Requirements of such adjuvant materials are: (1) that they be non-reactive with the other materials of our invention; (2) that they be organoleptically compatible with the other materials of our invention whereby the flavor of the ultimate consumable material to which the flavorings are added is not detrimentally affected by the use of the adjuvant; and (3) that they be ingestibly acceptable and thus nontoxic or otherwise non-deleterious. Apart from these requirements, conventional materials can be used and broadly include other flavor materials, vehicles, stabilizers, thickeners, surface active agents, conditioners and flavor intensifiers.

Such conventional flavoring materials include saturated fatty acids, unsaturated fatty acids and amino acids; alcohols including primary and secondary alcohols, esters, carbonyl compounds including ketones and aldehydes; lactones; other cyclic organic materials including benzene derivatives, alicyclic compounds, heterocyclics such as furans, pyridines, pyrazines and the like; sulfur-containing compounds including thiols, sulfides, disulfides and the like; proteins; lipids, carbohydrates; so-called flavor potentiators such as monosodium glutamate; magnesium glutamate, calcium glutamate, guanylates and inosinates; natural flavoring materials such as cocoa, vanilla and caramel; essential oils and extracts such as anise oil, clove oil and the like and artificial flavoring materials such as vanillin, ethyl vanillin and the like.

Specific preferred flavor adjuvants include but are not limited to the following: anise oil; ethyl-2-methyl butyrate; vanillin; cis-3-heptenol; cis-3-hexenol; trans-2-heptenal; butyl valerate; 2,3-diethyl pyrazine; methyl cyclopentenolone; benzaldehyde; valerian oil; 3,4-dimethoxyphenol; amyl acetate; amyl cinnamate; γ-butyryl lactone; furfural; trimethyl pyrazine; phenyl acetic acid; isovaleraldehyde; ethyl maltol; ethyl vanilin; ethyl valerate; ethyl butyrate; cocoa extract; coffee extract; peppermint oil; spearmint oil; clove oil; anethol; cardamom oil; wintergreen oil; cinnamic aldehyde; ethyl-2-methyl valerate; γ-hexenyl lactone; 2,4-decadienal; 2,4-heptadienal; methyl thiazole alcohol (4-methyl-5-β-hydroxyethyl thiazole); 2-methyl butanethiol; 4-mercapto-2-butanone; 3-mercapto-2-pentanone; 1-mercapto-2-propane; benzaldehyde; furfural; furfuryl alcohol; 2-mercapto propionic acid; alkyl pyrazine; methyl pyrazine; 2-ethyl-3-methyl pyrazine; tetramethyl pyrazine; polysulfides; dipropyl disulfide; methyl benzyl disulfide; alkyl thiophene; 2,3-dimethyl thiophene; 5-methyl furfural; acetyl furan; 2,4-decadienal; guiacol; phenyl acetaldehyde; β-decalactone; d-limonene; acetoin; amyl acetate; maltol; ethyl butyrate; levulinic acid; piperonal; ethyl acetate; n-octanal; n-pentanal; n-hexanal; diacetyl; monosodium glutamate; monopotassium glutamate; sulfur-containing amino acids, e.g., cysteine; hydrolyzed vegetable protein; 2-methylfuran-3-thiol;2-methyldihydrofuran-3-thiol; 2,5-dimethylfuran-3-thiol; hydrolyzed fish protein; tetramethyl pyrazine; propylpropenyl disulfide; propylpropenyl trisulfide; diallyl disulfide; diallyl trisulfide; dipropenyl disulfide; dipropenyl trisulfide; 4-methyl-2-[(methylthio)-ethyl]-1,3-dithiolane; 4,5-dimethyl-2-(methylthiomethyl)-1,3-dithiolne; and 4-methyl-2-(methylthiomethyl)-1,3-dithiolane. These and other flavor ingredients are provided in U.S. Patent Nos. 6,110,520 and 6,333,180.

The present invention may also be delivered in a liquid form. The range of the flavor / fragrance oil is from about 80 to about 99.5, and preferably from about 85 to about 95 weight percent. The cellulose polymer component, such as KLUCEL GF, is from about 0.5 to about 20, preferably from about 5 to about 15 and most preferably 3 to 8 weight percent of the liquid composition.

In a preferred embodiment the flavors system of the present invention is a liquid composition comprising from about 70 to about 97 weight percent flavor oil, from about 2 to about 30 weight percent emulsifier and cellulosic polymer of from about 0.5 to about 10 weight percent. More preferably the flavor oil is from about 78 to about 92 weight percent, the emulsifier level is from about 5 to about 20 weight percent and the cellulosic polymer is about 2-8 weight percent. Those with skill in the art will appreciate that depending on the cellulose material selected, the molecular weight of the cellulose and other factors, varying levels can be employed in the present invention. One of the key factors in the selection and level of the cellulose is the viscosity of the flavor oil mixture.

Further, among other considerations, the level of emulsifier used in the present invention is based on the miscibility of the cellulose in the flavor. The more miscible the cellulose is in the flavor oil the lower the emulsifier level required. Conversely, as the miscibility of the cellulose in the flavor decreases, additional emulsifier levels will be required. Other factors including flavor release, stability and processing ability will also determine the selection and the level of emulsifier used.

As noted above, the preferred emulsifier is CAPMUL MCM and the preferred cellulosic polymer is KLUCEL GF (Hercules Inc., Wilmington, DE). KLUCEL GF has a molecular weight (MW) of about 500,000 to about 1,500,000. Other suitable hydroxypropyl cellulose materials are described in U.S. 6,479,082, 5,128,155 and 4,259,355.

A further embodiment of the present invention is a chewing gum composition having a chewing gum base of about 90 to about 97 weight percent, a liquid flavor system of from about 1 to about 5 weight percent and a flavor containing particle of from about 1 to about 5 weight percent, wherein the flavor systems both contain a cellulose material. More specifically the present invention provides a gum base of about 95 to about 97 weight percent, a liquid flavor system of from about 1 to about 3 percent and a flavor containing particle of from about 1 to about 3.5 weight percent. The liquid flavor and the flavor particle both contain a cellulose material, preferably hydroxypropyl cellulose. In the liquid flavor system the ratio of the flavor oil to the cellulose material is greater than about 90, preferably about 95 to about 99 weight percent and most preferably about 98 weight percent and the cellulose material is from about less than about 10, preferably from about 1 to about 5 and most preferably about 4 weight percent in the liquid flavor oil that is incorporated in the chewing gum base. In addition to the liquid flavor, we have discovered that including a particle containing flavor and cellulose material improves the flavor delivery and intensity of the flavor. The preferred method of making the particle is extrusion. A preferred formulation for extrusion is flavor oil of from about 10 to about 30 weight percent, KLUCEL GF of from about 10 to about 45, preferably from about 25 to about 35 weight percent; emulsifier, preferably CAPMUL, of about 3 to about 7, preferably about 5 weight percent; silicon dioxide of from about 10 to about 20, more preferably about 15, mannitol of from about 20 to about 30 more preferably about 25 weight percent. The mixture is then preferably extruded to make the flavor particles that are included in the matrix of the chewing gum. The extruded material preferably has a milled particle size of about 200 to 500 microns.

The present invention also contemplates the use of liquid flavor systems containing a cellulose material in breath film applications. In addition to the base materials used to make breath films, such systems are disclosed in U.S. Patents 6,419,903, 5,409,715, 5,089,307 as well as U.S. Patent Applications 2003/0099692, 2003/0099691 and 2003/0035841. The cellulose material modifies the flavor as well as the release of flavor depending on the type of flavor oils used. For example, with a citrus flavored breath film both KLUCEL and ETHOCEL cellulose were effective in modifying the flavor delivery. When a menthol flavor was used in the breath film, it was noted that ETHOCEL suppresses the flavor, while KLUCEL intensifies the flavor.

The breath film comprises a breath film base and the liquid flavor system which in turn comprises a flavor oil from about 5 to about 13 weight percent, preferably from about 7 to about 12 and most preferably from about 8 to about 10 weight percent of the breath film. In the liquid flavor system for the breath film the cellulose material is included at a level of from about 0.1 to about 4 weight percent, preferably from about 0.3 to about 2 and most preferably about 0.5 to about 1.5 weight percent cellulose in the breath film.

The flavor products can be combined with one or more vehicles or carriers for adding them to the particular product. Vehicles can be edible or otherwise suitable materials such as ethyl alcohol, propylene glycol, water and the like, as described supra. Carriers include materials such as gum arabic, carrageenan, xanthan gum, guar gum and the like.

Optionally, additional agents may be added to achieve desired final particle characteristics. Additional agents include, but are not limited to, bulking carriers, solid flavors, solid fragrances, solid functional materials, fillers, and colorants. Bulking carriers may be added to increase apparent viscosity, increase bulk, provide structure in lower viscosity formulations such that a physically stable microparticle can form, and for other reasons. Bulking carriers include, among others, solid polyols, fats, dextrins, silicon dioxide, salts of fatty acids, calcium silicate, starches, and sugars. For example, dextrin and/or silicon dioxide may be added to aid in particle formation via increasing viscosity, although it should be noted that the presence of these fillers does not alter the fact that the fundamental characteristic of the particle remains the continuous flavor/fragrance - modified cellulose phase. The solid fillers and functional ingredients essentially are embedded as discrete entities in this continuous phase. Solid flavors or fragrances, such as spray dried products formed using gums, starches, and dextrins, may be added again to provide a particle about which the particle forms in lower viscosity formulations, and, of course, to provide enhanced flavor characteristics and release. These solid flavors and fragrances are often in the form of spray-dried flavors and fragrances, or may be high-intensity sweetener particles. Solid functional materials such as hydrocolloids and other water-soluble polymers may be added to lend certain desired characteristics to the microparticle, such as creating a viscous micro-environment in aqueous solutions which can enhance particle integrity and reduce flavor/fragrance diffusion. The level of the solid bulking and filling materials is from about 0.1 to about 70 weight percent, preferably from about 1 to about 50 weight percent and most preferably from about 5 to about 30 weight percent of the particle weight. The addition of solid fillers and bulking agents greatly increases the ability to make particles that can be processed under routine process conditions, such as extrusion.

Generally, the cellulosic material is dissolved in the flavor/fragrant chemical to achieve continuous-phase product of a desired viscosity, which is then subjected to particle size formation steps to achieve the desired final particle size. Suitable particle size formation steps include cutting, milling and chopping.

According to one embodiment of the invention, the cellulosic material is dissolved in the organic flavor/fragrance. This action may form a true solution, as noted by clarity and lack of a precipitate, or may result in a form that may more properly be referred to as a sol, or a somewhat imperfect solution. The term "solution" used throughout this specification is meant to encompass each of these situations. In fact, with higher cellulose contents, or via the addition of dispersed fillers as described above, the product may actually become a physical solid. This situation is also encompassed by the term "solution" since the continuous flavor/fragrance - modified cellulose phase still characterizes the particle. Regardless of its technical characterization, the product formed has a continuous-phase comprised of modified cellulose and flavor/fragrance. Continuous-phase is understood to mean that the cellulosic material and the flavor/fragrance materials form a phase which is throughout the particle. Additional agents may be dispersed in the continuous-phase as discrete particles suspended in the continuous phase without departing from the scope of the invention.

The additional agents may be mixed together with the cellulosic material and the flavor/fragrance to achieve the desired final characteristics. This may be done contemporaneously or as a separate step in the process.

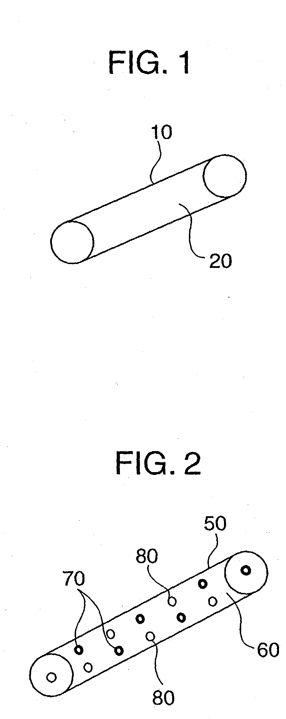

Referring to Figure 1, the particle 10 is depicted as made in a cylinder shape. A cylindrical shape is commonly achieved by extrusion processes. Figure 1 depicts the cellulose material and the flavor/fragrance material and any solvent as a continuous material 20, in a solid form or highly viscous material.

Referring to Figure 2, the particle 50 is depicted again in a cylindrical shape. The continuous phase comprises the cellulosic material and the flavor/fragrance and solvent 60 additionally contains various additive materials. The additive materials are depicted as darkened circles 70 and unshaded circles 80 to indicate different additive materials included in the continuous phase.

To achieve proper dissolution, heat may be applied as needed. Mild heating to about 60-70°C is generally sufficient to facilitate dissolution of the cellulose material in the flavor/fragrance in a reasonable time. The resultant solution is allowed to cool until it forms a continuous-phase product of the desired viscosity from thickened liquid to solid. If an emulsifier is to be added, the emulsifier should be premixed with the flavor/fragrance.

Depending upon the viscosity of this product, several particularizing techniques may be used. Any of the well-known or later developed particularization techniques may be used. Generally speaking, low viscosity products are not well-suited to techniques such as extrusion and milling. These techniques may, nonetheless, be used; they are simply not preferred for use with the lower viscosity products because they are harder to work with.

For liquid and semi-liquid and even semi-solid continuous-phase products, techniques well suited to fluid materials are preferred for the particularization step. Spray chilling, emulsification, and prilling are a few techniques suited to such lower viscosity starting materials. Further particularization may be achieved through other means, if necessary. When the cellulosic material is dissolved in the flavor/fragrance at levels below about 10% and more preferably between about 5-10%, the viscosity is potentially low enough to render the material suitable for these techniques. Cellulosic material content above this range generally yields a product that is more solid, and not generally suitable for these techniques. The end product of these techniques may be the final product or further treated by other particularization techniques and particularly those which were not available to the low viscosity product.

Continuous-phase products with higher viscosities, such as those containing greater than about 10% and preferably about 12-60% cellulosic material, are better suited to other techniques. Batch compounding, extrusion, milling, and compaction/granulation, and combinations thereof are better suited to the solid or semi-solid materials. The lower viscosity products may be used in such processes if the proper precautions, which are well-known to those skilled in these arts, are taken. These techniques are well-known in the field and, therefore, do not require discussion here.

One or more of these and other techniques may be used to render the continuous-phase product into appropriately sized particles for incorporation into the final product. Microparticle size can vary depending upon its final use, but generally ranges from about 10 to about 2000 microns and more preferably from about 500 to about 700 microns.

As noted above, when cellulosic material is chosen with dual solubility, such as hydroxypropyl cellulose, the resultant particles have some interesting characteristics. Because the cellulose is dissolved in the flavor/fragrance as a continuous-phase product, when the resultant microparticle is incorporated into an aqueous final product, the water-soluble hydroxypropyl cellulose at least partially migrates into its surroundings thereby releasing the flavor/fragrance. Nevertheless, the particles serve a valuable purpose because they are more stable and easier to handle than the flavor/fragrance chemicals themselves. Additionally, hydroxypropyl cellulose products of the current invention release slowly in hot aqueous solutions due to the fact that HPC is not soluble in water above approximately 45°C. Note that the same is true in solutions of other aqueous solutions containing ingredients which successfully compete for water against HPC. For example, a particle formed of HPC and flavor will not dissolve in an aqueous solution of 10% NaCl, although flavor chemicals will partition into the aqueous phase over time.

In another interesting embodiment, lower viscosity continuous-phase products may be particularized by encapsulation, for example, by spray drying. When the encapsulated product is introduced into a final product, depending upon the desired use, the encapsulant may disperse through the product, leaving only the flavor/fragrance particle of the semi-solid or semi-liquid cellulose-flavor/fragrance behind. Note that higher viscosity/solid particles formed by the current invention may be coated after formation using conventional techniques (e.g., fluid-bed coating) to further protect the flavor or fragrance. Suitable coatings may be formed from either hydrophilic (e.g., maltodextrin) or hydrophobic (e.g., waxes) coatings.

The inventive particles are suitable for incorporation into many products. Some non-limiting examples include food products and beverages, oral care products, cosmetics, detergents, shampoos, etc. As can be appreciated, some instances will lend themselves to the use of solid particles, while others will benefit from the addition of softer, semi-solid particles. Each of these situations can be achieved by the invention described herein. For example, a product such as toothpaste might be used in either category. The softer semi-solid particulate may be used to incorporate greater flavor while not adding abrasiveness. Similarly, some abrasive qualities might be desirable in toothpaste, and they could be introduced in the form of more solid particulates of the invention by including dispersed abrasive or polishing agents. Regardless, a wide variety of particles are disclosed herein for addressing a multitude of potential desired end products.

Unless noted to the contrary all percentages are given on a weight percent. The following examples are not meant to define or otherwise limit the scope of the invention. Rather the scope of the invention is to be ascertained according to the claims which follow the examples.

### EXAMPLE 1

| **HYDROXYPROPYL CELLULOSE EXTRUSION** | |
|---|---|
| **Ingredient** | **% By Weight** |
| Mannitol, powder | 67 |
| KLUCEL GF | 8 |
| Peppermint flavor | 25 |

### Procedure:

1. Add mannitol and KLUCEL to blender.
2. Mix for 5 minutes.
3. With blender running, add flavor to powder slowly.
4. Mix additional 5-8 minutes.
5. Mill product to remove lumps.
6. Add powder blend to extruder.
7. Reduce size of extrudate by milling.

| **Extrusion Conditions: (Werner & Pfleiderer ZSK Extruder)** | |
|---|---|
| Feed rate | 20 lbs/hr |
| Screw Speed | 400 RPM |
| Zone Temperatures (°C) | 0-0-90-90-90 |
| Die Block (°C) | 90 |
| Die Diameter | 1.6 mm |

### Results:

Product fed and extruded evenly, resulting in well formed strands. Strands were millable using dry ice to form small particulates.

When placed in toothpaste base, the particles retained a high level of flavor over several weeks of storage. Particles are soft enough to disintegrate during toothpaste use, thus releasing flavor into the oral cavity.

### EXAMPLE 2

| **HYDROXYPROPYL CELLULOSE EXTRUSION** | |
|---|---|
| **Ingredient** | **% By Weight** |
| Hydrophobic silicon dioxide, Syloid D11 | 10 |
| KLUCEL GF | 50 |
| Fragrance | 40 |

### Procedure:

1. Add Syloid and KLUCEL to blender.
2. Mix until uniform.
3. With blender running, add fragrance to powder slowly.
4. Mix until flowable.
5. Add powder blend to extruder.
6. Reduce size of extrudate by chopping.

| **Extrusion conditions: (Haake Lab Extruder)** | |
|---|---|
| Feed rate | Flood |
| Screw Speed | 50-60 RPM |
| Zone Temperatures (°C) | 70-85 |
| Die Block (°C) | 70-85 |
| Die Diameter | 1 mm |

### Results:

Product fed and extruded evenly, resulting in well formed strands. Strands were chopped by hand to small particulates. When placed in cold water, they dissolved slowly. In hot water, the particles retained their size and shape and did not dissolve appreciably over several hours.

### EXAMPLE 3

### BREATH FILMS

The following table of materials were mixed using a Waring laboratory blender:

| | **a** | **b** | **c** | **d** |
|---|---|---|---|---|
| **Ingredients** | **Parts** **(%)** | **Parts** **(%)** | **Parts** **(%)** | **Parts** **(%)** |
| Total Water | 77.98 | 77.98 | 77.93 | 77.70 |
| Cold Water | 46.79 | 46.79 | 46.76 | 46.62 |
| Hot Water | 31.19 | 31.19 | 31.17 | 31.08 |
| Sweetener(Ace-K/Sucralose) | 0.25 | 0.25 | 0.25 | 0.25 |
| Purity Gum 59 | 12 | 12 | 12 | 12 |
| HPMC E50 | 3 | 3 | 3 | 3 |
| KelGum GFS | 1 | 1 | 1 | 1 |
| Glycerine | 3 | 3 | 3 | 3 |
| TWEEN 80 | 0.25 | 0.25 | 0.25 | 0.25 |
| Menthol | 2.52 | -- | -- | -- |
| Menthol Mint | -- | 2.52 | -- | -- |
| 2% KLUCEL/98% Menthol Mint | -- | -- | 2.57 | -- |
| 10% ETHOCEL/90% Menthol Mint | -- | -- | -- | 2.8 |
| **TOTAL** | **100** | **100** | **100** | **100** |

Water (60%) was added first, followed by the sweeteners. Separately the gum and HPMC were mixed. The GFS gum, glycerine and TWEEN 80 were mixed separately. The flavors were added to the paste and mixed. The remaining water was heated to about 160°F and added to the blender at high speed. The contents were poured out of the blender and spread to the desired thickness of about 0.2 millimeters.

The flavor of the films containing either 9% menthol or menthol mint was tested by a panel of tasters and the results were as follows:
a. menthol was found to be much too strong, and burning.
b. menthol mint was found to have a strong flavor.
c. menthol mint / KLUCEL was found to have an enhanced flavor.
d. menthol mint / ETHOCEL was found to have a delayed flavor onset, with a slow gradual release which was found to be pretty strong over time.

Overall the effect of the KLUCEL and ETHOCEL material were to either enhance or suppress the effect of the menthol. In the case of ETHOCEL, delayed onset allowed a higher flavor loading to be more palatable.

### Example 4

### Chewing Gum

The following ingredients were made into chewing gum products:

| **Ingredients** | **Control (%)** | **Flavor System 1 (%)** |
|---|---|---|
| Unflavored Gum Base | 98 | 95.5 |
| Wintergreen Flavor | 2 | 2 |
| Wintergreen/KLUCEL Extrudate | - | 2.5 |
| **TOTAL** | **100** | **100** |

In the Control sample, wintergreen flavor was added neat. In Flavor System 1, the total wintergreen flavor added was 2.5%, of which 0.5% was added in the form of extruded particulates similar to those formed in Example 1, containing 20% wintergreen flavor, 60% KLUCEL and the remainder filler.

A panel was asked to evaluate the flavor of the gums over a period of time on a scale of 0 (no flavor) to 5 (highest flavor).

| **Material** | **Time (Minutes)** | **Flavor Intensity** |
|---|---|---|
| Control | 0.5 | 4 |
| With Extrudate | 0.5 | 4.1 |
| Control | 1.5 | 3.9 |
| With Extrudate | 1.5 | 3.9 |
| Control | 3 | 3.3 |
| With Extrudate | 3 | 3.4 |
| Control | 5 | 2.4 |
| With Extrudate | 5 | 3.1 |
| Control | 10 | 1.8 |
| With Extrudate | 10 | 2.4 |
| Control | 15 | 1.3 |
| With Extrudate | 15 | 2.0 |

This example indicates that the addition of the extruded flavor / HPC material is able to provide a longer lasting, more intense flavor than flavors that do not contain the extrudate material.

A similar test was conducted using a strawberry flavor. The formulation of the products is provided below.

| **Ingredients** | **Control (%)** | **Flavor System 1 (%)** |
|---|---|---|
| Unflavored Gum Base | 99 | 98.96 |
| Strawberry Flavor | 1 | - |
| Strawberry/KLUCEL mixture (96.5/3.5) | - | 1.04 |
| **TOTAL** | **100** | **100** |

The total strawberry flavor in both gum samples was 1%. In Flavor System 1, the flavor was added as a homogeneous mixture.

The chewing gum was again evaluated by a panel for flavor intensity over time on a scale of 0 (no flavor) to 9 (highest flavor):

| **Material** | **Time (Minutes)** | **Flavor Intensity** |
|---|---|---|
| Control | 0.5 | 7.0 |
| Polymer Mixture | 0.5 | 6.9 |
| Control | 1 | 6.3 |
| Polymer Mixture | 1 | 6.3 |
| Control | 2 | 5.6 |
| Polymer Mixture | 2 | 5.7 |
| Control | 5 | 3.6 |
| Polymer Mixture | 5 | 3.9 |
| Control | 7 | 2.9 |
| Polymer Mixture | 7 | 3.3 |
| Control | 10 | 2.2 |
| Polymer Mixture | 10 | 2.9 |
| Control | 12 | 2.0 |
| Polymer Mixture | 12 | 2.3 |

The above data indicates that the use of the flavor and cellulose mixture improved the flavor intensity over time in a chewing gum product especially after 5 to 12 minutes of chewing.

As noted above, the above description is a non-limiting example of certain preferred particles of the invention and methods for making them. Other obvious variants will be apparent and to those skilled in the art without parting from the scope and spirit of the invention as claimed herein.

## Claims

1. A particle composition comprising:
from 0.5 to 95 weight % organically soluble cellulosic material selected from the group consisting of ethyl cellulose and hydroxypropyl cellulose dissolved in
from 5 to 99.5% organic solvent selected from organic fragrance chemicals, and/or organic flavor chemicals,
wherein said particles are approximately from 1 to 2000 microns in size.

2. The particle composition of claim 1 further comprising 1-70% of triglyceride oil.

3. The particle composition of claim 1 or claim 2, having a viscosity before particle formation ranging from a thickened liquid to substantially solid material.

4. The particle composition as claimed in any one of claims 1 to 3 wherein said particles are from 500 to 700 microns in size.

5. The composition of any one of claims 1 to 5 further comprising 1-95% of at least one agent selected from the group consisting of solid bulking carriers, solid flavors, solid fragrances, solid functional materials, and colorants.

6. The composition of claim 4 further comprising liquid bulking carriers and liquid functional filler materials.

7. The particle composition of any one of claims 1 to 6 wherein the cellulosic content is below about 15%, has a viscosity of from 100 to 20,000 mPas and further comprising water soluble encapsulant surrounding said particle.

8. The particle composition of any one of claims 1 to 8 wherein said particles comprise approximately from 5 to 25 weight % cellulosic material.

9. The particle composition of claim 8 wherein said particles comprise from 5 to 15 weight % cellulosic material.

10. The particle composition of any one of claims 1 to 7 wherein said particles comprise greater than approximately 25% cellulosic material.

11. The particle composition of claim 10 wherein said particles comprise approximately from 20 to 50% cellulosic material.

12. A method of producing particles comprising the steps of:
dissolving, with or without heat as needed, an organically compatible cellulosic material in an organic solvent selected from the group consisting of triglycerides, a flavor material and a fragrance material;
allowing the resultant mixture to equilibrate to a continuous phase product having a desired final viscosity from a thickened liquid to substantially solid; and
forming said continuous phase product into particles of 1-2000 microns in size.

13. The method of claim 12, further comprising the steps of:
incorporating said particles into a foodstuff or a cosmetic product.

14. The method of claim 12 wherein additional agents selected from the group consisting of bulking carriers, solid flavors, solid fragrances, solid functional materials, fillers and colorants are added during formation of the continuous phase product.

15. The method of claim 11 wherein said particularizing step is carried out by one or more of the techniques selected from the group consisting of extrusion, milling, compaction, granulation, spray chilling, emulsification, spray drying, and prilling.

16. The method of claim 12 further comprising coating the particle with a hydrophilic or hydrophobic coating.

17. A liquid flavor system comprising from 80 to 99.5 weight percent flavor oil and from 0.5 to 20 weight percent cellulose polymer.

18. A liquid flavor or fragrance system comprising:
from 70 to 97 weight percent flavor oil;
from 2 to 30 weight percent emulsifier; and
from 0.5 to 10 weight percent cellulose polymer.

19. The liquid flavor or fragrance system of Claim 17 wherein the cellulose polymer is selected from the hydroxypropyl cellulose and ethyl cellulose.

20. The liquid flavor or fragrance system of Claim 18 wherein the emulsifier is selected from mono and di-glycerol esters of fatty acids, polyglycerol esters and sorbitol esters.

21. A chewing gum comprising:
a chewing gum base;
liquid flavor oil of from 1 to 5 weight percent of the chewing gum; and
a liquid flavor system of 1 to 5 weight percent of the chewing gum, further provided that the liquid flavor system is comprised of greater than 90 weight percent flavor oil and less than 10 weight percent cellulose polymer.

22. The chewing gum of Claim 21 wherein the liquid flavor system further comprises an emulsifier.

23. A breath film comprising:
a breath film base material; and
a liquid flavor system of from 5 to 13 weight percent of the breath film;
wherein the liquid flavor system, a cellulosic polymer is provided at a level from 0.1 to 4 weight percent of the breath film.

24. The breath film of Claim 22 wherein the cellulose material is hydroxypropyl cellulose.
